# EUROPÄISCHE PATENTSCHRIFT

(11) **EP 1 382 319 B1**
(45) Veröffentlichungstag und Bekanntmachung des Hinweises auf die Patenterteilung: **15.10.2008**
(21) Anmeldenummer: 03015740.8
(22) Anmeldetag: 10.07.2003
(51) Int. Cl.: A61F 17/00

(54) **Buchartig auf- und zuklappbarer Verbandskasten**
Booklike open- and closable first-aid container
Trousse de premiers secours ressemblant à un livre

(30) Priorität: 15.07.2002 DE 20211639 U
(43) Veröffentlichungstag der Anmeldung: 21.01.2004
(73) Patentinhaber: Leina-Werke GmbH, 51570 Windeck-Rosbach (DE)
(72) Erfinder: Steinhauer, Hartmut, 57537 Forst (DE)
(74) Vertreter: Herden, Andreas F.

(56) Entgegenhaltungen:
- EP-A- 0 375 925
- EP-A- 0 480 532
- EP-A- 1 284 124
- DE-U- 29 519 064
- US-A- 369 438
- US-A- 1 520 444

## Beschreibung

Die Erfindung betrifft einen buchartig auf- und zuklappbaren Verbandkasten.

Buchartig auf- und zuklappbare Behältnisse für Verbandmaterial sind bekannt. So wird ein solcher Behälter in FR 1095236 (Deckers) beschrieben, der wie ein Buch oder eine Mappe aufklappbar ist, und Fächer zur Aufnahme von Verbandzeug und ähnlichem aufweist.
Eine buchartig aufklappbare "Nothilfepackung bestehend aus einer flexiblen Materialbahn" wird in DE 9316351 (Kalff) beschrieben.
Auch die Nothilfepackung gemäß EP 1174105 (Kalff) ist "buchartig zusammenklappbar" und weist einen flexiblen Rücken auf.
Die DE 295 19 054 zeigt einen auffaltbaren Nothilfebehälter. Die GB 2 229 709 A zeigt eine buchartig aufklappbare Box mit Flügeln an den Seitenwänden. Vorteilhaft an diesen aufklappbaren Behältnissen ist gegenüber einem Verbandkasten mit einem Deckel, dass in aufgeklappten Zustand das Verbandzeug übersichtlicher darin liegt und besser zugänglich ist.
Nachteilig an den bekannten buchartig aufklappbaren Behältnissen ist, dass sie wegen eines Rückenteils aus flexiblen Material leicht zusammendrückbar und stoßempfindlich sind. So sind diese Behältnisse beispielsweise für die Aufbewahrung im Kofferraum eines Kraftfahrzeuges weniger gut geeignet.
Der Erfindung liegt demgegenüber die Aufgabe zugrunde, einen Kasten zu schaffen, der zum einen buchartig auf-und zuklappbar ist, und zum anderen sich nicht zusammendrücken lässt.

Diese Aufgabe wird nach der Erfindung gemäß den Merkmalen des Anspruch 1 dadurch gelöst, dass der Verbandkasten ein Rückenteil aus steifen Material hat, an dessen Längsseiten zwei Seitenteile klappbar angebracht sind. Die Seitenteile haben randseitige Wände und bilden so zwei wannenartige Hälften, in denen das Verbandzeug untergebracht werden kann. Das steife Rückenteil verhindert ein Zusammendrücken des Kastens, und damit eine mögliche Beschädigung des Verbandzeuges. Durch Scharniere an beiden Längsseiten des Rückenteils wird erreicht, dass der Verbandkasten wie eine Mappe in zwei Hälften aufklappbar ist.

Vorteilhafte und bevorzugte Weiterbildungen sind den jeweils zugeordneten Unteransprüchen zu entnehmen.

Nach einer zweckmäßigen Ausführungsform der Erfindung haben die Seitenteile nur an den nicht mit dem Rückenteil verbundenen Seiten eine Wand, was den Vorteil hat, dass so in geöffnetem Zustand Rückenteil und Seitenteile eine durchgehende Fläche bilden.

Eine weitere zweckmäßige Ausführungsform hat in etwa gleich hohe Seitenwände, so dass in jeder Hälfte des Verbandkasten etwa gleich viel Verbandzeug untergebracht werden kann. Auch eine leicht unterschiedliche Wandhöhe je Seite, so etwa 2/3 zu 1/3 ist je nach gewünschter Verteilung des Verbandzeuges auf die zwei Hälften denkbar. Ferner ist denkbar, nur eine Seite mit Wänden zu versehen, deren Höhe etwa der Breite der Rückenteile entspricht.
Bei einer weiteren Ausführungsform bestehen auch die Seitenteile aus steifem Material. So ist der gesamte Kasten gegen von außen einwirkende Stöße unempfindlich und eignet sich so insbesondere für den Einsatz im Kraftfahrzeug.

Bei einer Weiterbildung der Erfindung sind Rückenteil und Seitenteile mit Filmscharnieren verbunden, die eine dünne Außenwand ermöglichen und sehr kompakt sind.
Werden, wie bei einer weiteren Ausführungsform vorgesehen, Filmscharniere angebracht, die über die gesamte Seitenlänge reichen, hat dies den Vorteil, dass in geschlossenem Zustand keine Spalten, durch die Schmutz und Feuchtigkeit eindringen kann, vorhanden sind.

Nach einer Weiterbildung der Erfindung ist der Kasten mit Clipverschlüssen verschließbar. Diese Clipverschlüsse sind zum einen sehr kompakt und zum anderen billig herstellbar.

Eine Ausführungsform hat nur einen Clipverschluss, der mittig angebracht ist. So lässt sich der Verbandkasten leicht und nur mit einem Handgriff öffnen.

Bei einer Ausführungsform hat auch das Rückenteil an seinen Querseiten randseitige Wände, die etwa die Höhe der Seitenteile haben. Im geschlossenen Zustand überschneiden sich die Wände des Rückenteils mit denen der Seitenteile. Im geöffneten Zustand hat der Kasten dann rundherum eine Wand. So wird das Verbandmaterial am Herausfallen gehindert.

Bei einer weiteren Ausführungsform sind die Wände der Seitenteile so geformt, dass sie in geschlossenen Zustand auf einer Ebene mit den Stegen des Rückenteiles liegen. Dies kann entweder durch weiter innen liegende Wände am Rückenteil, oder durch Wände an den Seitenteilen, die im Bereich der im geschlossenen Zustand an die Wände des Rückenteils grenzenden Fläche weiter innen liegen, erreicht werden. So bildet der Kasten einen gut unterbringbaren und gut stapelbaren Quader. Nach einer zweckmäßigen Ausführungsform besteht der Verbandkasten aus nur einem Stück. Ein solcher Kasten kann kostengünstig z.B. aus Kunststoff im Spritzgussverfahren, vorzugsweise aus Polypropylen, hergestellt werden. Außerdem wird so eine minimale Masse erreicht.

Bei einer weiteren Ausführungsform überschneiden sich die Wände in geschlossenem Zustand und haben im Bereich der Überschneidung eine treppenartig ineinandergreifende Form. So ist der Verbandkasten noch besser gegen Nässe und Schmutz geschützt.

Nach einer zweckmäßigen Ausführungsform hat das Rückenteil des Verbandkasten eine nach innen gewölbte Form, so wird eine höhere Steifigkeit erreicht.

Nach einer Ausführungsform hat das Rückenteil kurze Stege, an denen die Seitenteile beweglich befestigt sind. So stoßen die querseitigen Wände der Seitenteile auch in geschlossenem Zustand nicht gegen das Rückenteil.

Nach einer Weiterbildung der Erfindung sind am Rückenteil kurze Stege angebracht, die in geschlossenem Zustand an die Wände der Seitenteile grenzen. So haben die Seitenteile beim Zuklappen des Verbandkasten einen definierten Anschlagpunkt.

Dadurch bleibt der Verbandkasten beim Zuklappen symmetrisch. So wird erreicht, dass bei einem Verbandkaten mit Verschluss die beiden Teile des Verschlusses bei Zuklappen immer aufeinander passen.

Bei einer zweckmäßige Ausführungsform der Erfindung haben die Wände des Rückenteils jeweils eine Versteifungsrippe. So sind die Wände stabiler.

In folgendem soll die Erfindung an einem Ausführungsbeispiel anhand Fig. 1 bis Fig. 4 erläutert werden.
- Fig. 1: zeigt den Verbandkasten in der Draufsicht von oben ;
- Fig. 2: zeigt den Verbandkasten in der Draufsicht von vorne;
- Fig. 3: zeigt den Verbandkasten in der Draufsicht von der Seite;
- Fig. 4: zeigt den Verbandkasten in der Draufsicht von vorne im geöffneten Zustand;
- Fig. 5: zeigt eine schematische Schnittansicht des Verbandkasten entlang der Linie A-A aus Fig.1 in aufgeklapptem Zustand.

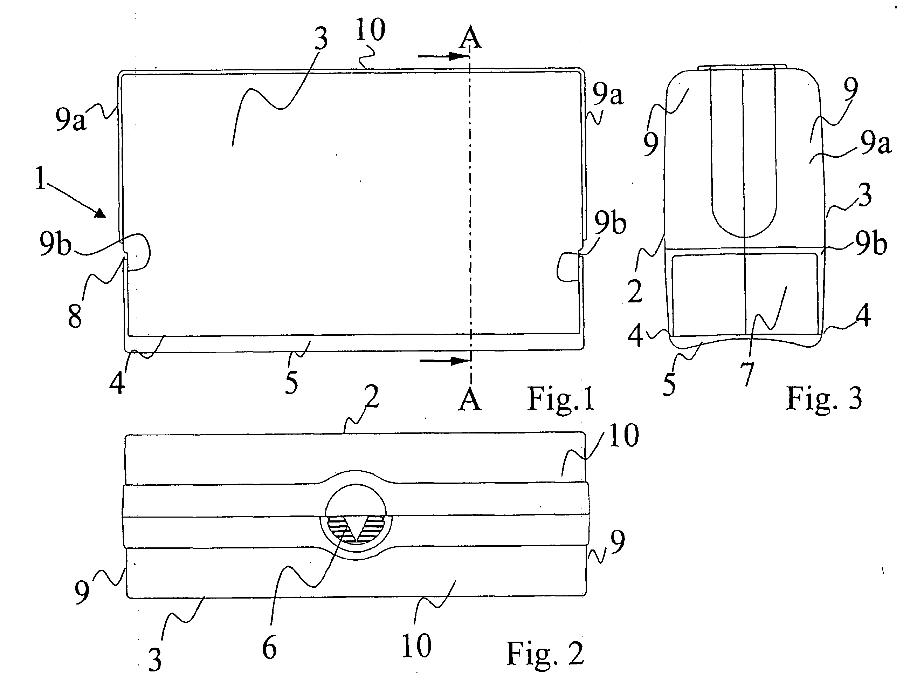

Fig. .1 zeigt den erfindungsgemäßen Verbandkasten 1 von oben. Zu sehen ist das obere Seitenteil 3, das steife Rückenteil 5 und ein über die gesamte Längsseite reichender Filmscharnier 4. Rückenteil 5 und Seitenteile 3,2(erkennbar in Fig. 2) sind durch die Filmscharniere klappbar verbunden. Das Rückenteil 5 ist an seinen Querseiten mit randseitigen Wänden 7 (zu sehen in Fig. 3) versehen, die in offenem Zustand etwa die Höhe der Wände 9 der Seitenteile 2,3 haben, und in geschlossenem Zustand mit den randseitigen Wänden 9 der Seitenteile 2,3 etwa auf einer Ebene liegen, wie an der Grenzlinie 8 zu erkennen ist. Durch die randseitigen Wände 9,10 bilden Rückenteil 5 und Seitenteile 2,3 eine wannenartige Form.

Fig. 2 zeigt den Verbandkasten von vorne in geschlossenem Zustand. Zu erkennen ist der mittige Clipverschluss 6 und die Seitenteile 2,3 mit den randseitigen Wänden 9,10.

Fig. 3 zeigt den Verbandkasten von der Seite. Hier sind die Seitenteile 2,3 zu erkennen. Diese sind durch zwei Filmscharniere 4 an den Längsseiten des Rückenteils angeschlagen. So kann der Verbandkasten in zwei Hälften aufgeklappt werden. Dabei klappen die beiden Seitenteile 9 vom Rückenteil 5 nach beiden Seiten mit den Filmscharnieren 4 als Drehpunkt weg. Die randseitige Wand 7 des Rückenteils überschneidet sich mit den Wänden 9 der Seitenteile. Bei diesem Ausführungsbeispiel überlappen sich in geschlossenem Zustand des Verbandkastens Wandabschnitte 9b der Seitenteile 9, die im Überschneidungsbereich (gesamte Wand 7 des Rückenteils) weiter innen liegen als die Wände des Rückenteils, so dass die Wände 7 des Rückenteils und nicht überlappenden Wandabschnitte 9a der Seitenteile 9 in etwa zueinander fluchten.

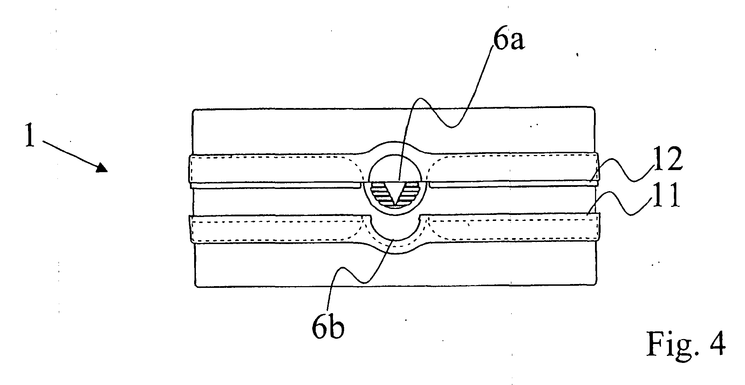

Fig. 4 zeigt den Verbandkasten in nicht geschlossenem Zustand. Man sieht die beiden ineinander passenden Teile des Clipverschlusses 6a,6b. Die randseitigen Wände der Seitenteile sind so geformt, dass sie sich in geschlossenem Zustand etwas überschneiden und durch aufeinander passende Stufen 11,12 ineinander greifen. So wird der Verbandkasten vor Schmutz und Nässe geschützt.

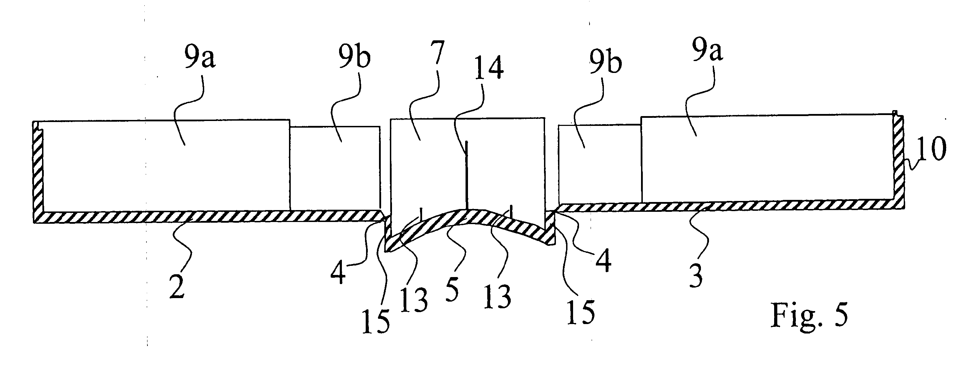

Fig. 5 zeigt eine schematische Schnittansicht des Verbandkasten entlang der Linie A-A aus Fig. 1, aber nicht in geschlossenem, sondern im aufgeklappten Zustand. Die Seitenteile 2,3 sind über die Filmscharniere 4 nach beiden Seiten aufgeklappt. Das Rückenteil 5 hat eine nach innen gebogene Form und an seiner Längsseite kurze Stege 15, an denen die Seitenteile 2,3 mit den Filmscharnieren 4 beweglich befestigt sind. Die Wand 7 des Rückens 5 hat kurze Stege 13, an denen die überlappenden Wandabschnitte 9d in geschlossenem Zustand anstoßen. Außerdem hat die Wand 7 des Rückens 5 zur Verstärkung eine innenseitige, mittige Versteifungsrippe 14. Durch Aussparungen der Seitenwände 9 des der Seitenteile 1, 3 in den Wandabschnitten 9b stoßen diese auch in geschlossenem Zustand nicht an die Versteifungsrippe 14.

## Patentansprüche

1. Buchartig auf- und zuklappbarer Verbandkasten (1) mit einem Rückenteil (5) und zwei an dem Rückenteil befestigten Seitenteilen (2,3),
wobei
das Rückenteil (5) steif ausgebildet ist, die Seitenteile (2,3) an den beiden Längsseiten des Rückenteils beweglich befestigt sind und die Seitenteile randseitig Wände (9,10) haben,
wobei das Rückenteil (5) an seinen Querseiten randseitige Wände (7) hat, die etwa die Höhe der Wände (9,10) der Seitenteile haben und die in geschlossenem Zustand mit Wandabschnitten (9b) der Seitenteile überlappen,
**dadurch gekennzeichnet, dass** die Wandabschnitte der Seitenteile (9) im Überschneidungsbereich (9b) weiter innen liegen als die Wände (7) des Rückenteils, so dass die Wände (7) des Rückenteils und die nicht überlappenden Wandabschnitte (9a) der Seitenteile (9b) in etwa zueinander fluchten.

2. Verbandkasten nach Anspruch 1, **dadurch gekennzeichnet, dass** ein oder beide Seitenteile an den nicht an das Rückenteil grenzenden Seiten eine Wand haben.

3. Verbandkasten nach Anspruch 1 oder 2, **dadurch gekennzeichnet, dass** die Wände der Seitenteile in geschlossenem Zustand etwa bis zur Mitte des Rückenteils reichen.

4. Verbandkasten nach einem der vorhergehenden Ansprüche, **dadurch gekennzeichnet, dass** die Seitenteile aus steifem Material bestehen.

5. Verbandkasten nach einem der vorhergehenden Ansprüche, **dadurch gekennzeichnet, dass** Rückenteil und Seitenteile mit Filmscharnieren (4) verbunden sind.

6. Verbandkasten nach einem der vorhergehenden Ansprüche, **dadurch gekennzeichnet, dass** Rückenteil und Seitenteile mit je einem, etwa über die gesamte Längsseite reichenden, Filmscharnier (4) versehen sind.

7. Verbandkasten nach einem der vorhergehenden Ansprüche, **dadurch gekennzeichnet, dass** der Verbandkasten an den Wänden der Seitenteile verschließbar ist.

8. Verbandkasten nach einem Anspruch 7, **dadurch gekennzeichnet, dass** der Verbandkasten mit einem oder mehreren Clipverschlüssen verschließbar ist.

9. Verbandkasten nach Anspruch 8, **dadurch gekennzeichnet, dass** der Verbandkasten an der Längsseite mittig einen Clipverschluss (6) aufweist.

10. Verbandkasten nach einem der vorhergehenden Ansprüche, **dadurch gekennzeichnet, dass** der Verbandkasten einstückig ist.

11. Verbandkasten nach einem der vorhergehenden Ansprüche, **dadurch gekennzeichnet, dass** der Verbandkasten aus Polypropylen besteht.

12. Verbandkasten nach einem der vorhergehenden Ansprüche, **dadurch gekennzeichnet, dass** sich die Wände der Seitenteile (9) im geschlossenen Zustand etwas überschneiden und durch randseitig aufeinander passende Stufen (11,12) ineinandergreifen.

13. Verbandkasten nach einem der vorhergehenden Ansprüche, **dadurch gekennzeichnet, dass** das Rückenteil (5) eine nach innen gewölbte Form hat.

14. Verbandkasten nach einem der vorhergehenden Ansprüche, **dadurch gekennzeichnet, dass** das Rückenteil (5) an seinen Längsseiten Stege (15) aufweist, an denen die Seitenteile (2,3) beweglich befestigt sind.

15. Verbandkasten nach einem der vorhergehenden Ansprüche, **dadurch gekennzeichnet, dass** die Wände (7) des Rückenteils (5) Stege (13) haben, an denen die Wände (9) der Seitenteile in geschlossenem Zustand anstossen.

16. Verbandkasten nach einem der vorhergehenden Ansprüche, **dadurch gekennzeichnet, dass** die Wände (7) des Rückenteils (5) wenigstens eine Versteifungsrippe (14) haben.

17. Buchartig auf- und zuklappbarer Verbandkasten (1) mit einem Rückenteil (5) und zwei an dem Rückenteil befestigten Seitenteilen (2,3),
wobei
das Rückenteil (5) steif ausgebildet ist, die Seitenteile (2,3) an den beiden Längsseiten des Rückentéils beweglich befestigt sind und die Seitenteile randseitig Wände (9,10) haben,
**dadurch gekennzeichnet, dass** das Rückenteil (5) eine nach innen gewölbte Form hat.

## Claims

1. A first aid kit (1), which may be opened and closed like a book, having a rear component (5) and two side components (2,3) attached to the rear component,
wherein
the rear component (5) is constructed stiff, the side components (2, 3) are movably fixed to the two longitudinal sides of the rear component, and the side components have walls (9, 10) at the border-side,
wherein the rear component (5) has border-sided walls (7) at its transverse sides, which walls approximately have the height of the walls (9, 10) of the side components, and which in closed condition, overlap with wall sections (9b) of the side components,
**characterized in that** in the overlapping region, the wall sections of the side components (9) are more inside than the walls (7) of the rear component so that the walls of the rear component and the non-overlapping wall sections (9a) of the side components (9b) approximately align with each other.

2. The first aid kit according to claim 1, **characterized in that** one or both side components have a wall at the sides not adjoining the rear component.

3. The first aid kit according to claim 1 or 2, **characterized in that** in closed condition, the walls of the side walls approximately range to the mid of the rear component.

4. The first aid kit according to one of the preceding claims, **characterized in that** the side components consist of stiff material.

5. The first aid kit according to one of the preceding claims, **characterized in that** the rear component and the side components are connected by means of film hinges (4).

6. The first aid kit according to one of the preceding claims, **characterized in that** the rear component and the side components each are connected by means of a film hinge approximately ranging over the whole longitudinal side.

7. The first aid kit according to one of the preceding claims, **characterized in that** the first aid kit is closable at the walls of the side components.

8. The first aid kit according to claim 7, **characterized in that** the first aid kit is closable by means of one or more clip latches.

9. The first aid kit according to claim 8, **characterized in that** the first aid kit has a clip latch (6) in the mid of the longitudinal side.

10. The first aid kit according to one of the preceding claims, **characterized in that** the first aid kit consists of one piece.

11. The first aid kit according to one of the preceding claims, **characterized in that** the first aid kit consists of polypropylene.

12. The first aid kit according to one of the preceding claims, **characterized in that** in closed condition, the walls of the side components (9) coincide a little bit, and mate by means of ladders (11, 12) matching on top of each other at the border-side.

13. The first aid kit according to one of the preceding claims, **characterized in that** the rear component (5) has a form being cambered inwards.

14. The first aid kit according to one of the preceding claims, **characterized in that** at its longitudinal sides, the rear component (5) has bars (15), to which the side components are movably attached.

15. The first aid kit according to one of the preceding claims, **characterized in that** the walls (7) of the rear component (5) have bars (13), against which the walls (9) of the side components abut in closed condition.

16. The first aid kit according to one of the preceding claims, **characterized in that** the walls (7) of the rear component (5) have at least one strengthening rib (14).

17. A first aid kit (1), which may be opened and closed like a book, having a rear component (5) and two side components (2, 3) attached to the rear component,
wherein
the rear component (5) is constructed stiff, the side components (2, 3) are movably attached at the two longitudinal sides of the rear component, and the side components have walls (9, 10) at the border-side,
**characterized in that** the rear component (5) has a form being cambered inwards.

## Revendications

1. Trousse de premier secours (1) pouvant s'ouvrir et se fermer à la façon d'un livre comprenant une partie arrière (5) et deux parties latérales (2, 3) fixées sur la partie arrière,
la partie arrière (5) étant conçue rigide, les parties latérales (2, 3) étant fixées de façon mobile sur les deux grands côtés de la partie arrière et les parties latérales présentant des parois (9, 10) côté bordure,
la partie arrière (5) présentant sur ses côtés transversaux des parois (7) côté bordure qui ont à peu près la hauteur des parois (9, 10) des parties latérales et qui, à l'état fermé, viennent en superposition avec les parties de paroi (9b) des parties latérales,
**caractérisée en ce que** les tronçons de paroi des parties latérales (9) dans la zone de chevauchement (9b) se situent davantage à l'intérieur que les parois (7) de la partie arrière, de sorte que les parois (7) de la partie arrière et les tronçons de paroi (9a) des parties latérales (9b) qui ne viennent pas en superposition sont à peu près alignés entre eux.

2. Trousse de premier secours selon la revendication 1, **caractérisée en ce qu'**une partie latérale ou les deux parties latérales présentent une paroi sur les côtés non contigus à la partie arrière.

3. Trousse de premier secours selon la revendication 1 ou 2, **caractérisée en ce que** les parois des parties latérales vont à peu près jusqu'au centre de la partie arrière dans l'état fermé.

4. Trousse de premier secours selon l'une quelconque des revendications précédentes, **caractérisée en ce que** les parties latérales sont à base de matériau rigide.

5. Trousse de premier secours selon l'une quelconque des revendications précédentes, **caractérisée en ce que** la partie arrière et les parties latérales sont reliées avec des charnières en film (4).

6. Trousse de premier secours selon l'une quelconque des revendications précédentes, **caractérisée en ce que** la partie arrière et les parties latérales sont dotées chacune d'une charnière en film (4) s'étendant à peu près sur l'ensemble du grand côté.

7. Trousse de premier secours selon l'une quelconque des revendications précédentes, **caractérisée en ce que** la trousse de premier secours peut être fermée sur les parois des parties latérales.

8. Trousse de premier secours selon la revendication 7, **caractérisée en ce que** la trousse de premier secours peut être fermée avec une ou plusieurs fermetures à clips.

9. Trousse de premier secours selon la revendication 8, **caractérisée en ce que** la trousse de premier secours présente sur le grand côté au centre une fermeture à clips (6).

10. Trousse de premier secours selon l'une quelconque des revendications précédentes, **caractérisée en ce que** la trousse de premier secours est d'une seule pièce.

11. Trousse de premier secours selon l'une quelconque des revendications précédentes, **caractérisée en ce que** la trousse de premier secours est en polypropylène.

12. Trousse de premier secours selon l'une quelconque des revendications précédentes, **caractérisée en ce que** les parois des parties latérales (9) viennent quelque peu en superposition dans l'état fermé et s'emboîtent les unes dans les autres par des niveaux (11, 12) adaptés les uns aux autres côté bordure.

13. Trousse de premier secours selon l'une quelconque des revendications précédentes, **caractérisée en ce que** la partie arrière (5) a une forme incurvée vers l'intérieur.

14. Trousse de premier secours selon l'une quelconque des revendications précédentes, **caractérisée en ce que** la partie arrière (5) présente sur ses grands côtés des barrettes (15) sur lesquelles les parties latérales (2, 3) sont fixées de façon mobile.

15. Trousse de premier secours selon l'une quelconque des revendications précédentes, **caractérisée en ce que** les parois (7) de la partie arrière (5) présentent des barrettes (13), sur lesquelles les parois (9) des parties latérales butent dans l'état fermé.

16. Trousse de premier secours selon l'une quelconque des revendications précédentes, **caractérisée en ce que** les parois (7) de la partie arrière (5) ont au moins une nervure de renfort (14).

17. Trousse de premier secours (1) pouvant être ouverte et fermée à la façon d'un livre avec une partie arrière (5) et deux parties latérales (2, 3) fixées sur la partie arrière,
la partie arrière (5) étant conçue rigide, les parties latérales (2, 3) étant fixées de façon mobile sur les deux grands côtés de la partie arrière et les parties latérales ayant des parois (9, 10) côté bordure,
**caractérisée en ce que** la partie arrière (5) a une forme incurvée vers l'intérieur.
